# EUROPEAN PATENT APPLICATION

(11) **EP 4 310 076 A1**
(43) Date of publication of application: **24.01.2024**
(21) Application number: 22807635.2
(22) Date of filing: 19.04.2022
(51) Int. Cl.: C07D 207/16, A61K 31/40, A61P 17/00, A61P 17/06, A61P 17/04, A61P 11/00

(54) **COMPOUND COMPRISING PYRROLIDINE AS LINKER, AND PHARMACEUTICAL COMPOSITION COMPRISING SAME**

(30) Priority: 11.05.2021 KR 20210060990
(71) Applicant: Bead Tech Inc., Ansan-si, Gyeonggi-do 15610 (KR); Sogang University R&DB Foundation, Seoul 04107 (KR)
(72) Inventor: LEE, Yoon Sik, Anyang-si Gyeonggi-do 13932 (KR); KIM, Seok In, Yongin-si Gyeonggi-do 16951 (KR); JU, Bong Gun, Seoul 02786 (KR); JEONG, Ha Yan, Seoul 04726 (KR); KONG, Jun Soo, Suwon-si Gyeonggi-do 16628 (KR); KIM, Myeongok, Suwon-si Gyeonggi-do 16668 (KR)
(74) Representative: Prüfer & Partner mbB Patentanwälte · Rechtsanwälte
(86) International application number: PCT/KR2022/005569
(87) International publication number: WO 2022/239987

(57) **Abstract**

The present invention relates to a compound containing a pyrrolidine moiety as a linker or a pharmaceutically acceptable salt thereof. The present invention also relates to a composition for preventing and treatment of the respiratory diseases or the skin conditions such as atopy and psoriasis and bronchial diseases, including the compound as an active ingredient.

## Description

### Technical Field

The present invention relates to a composition that can prevent or treat skin diseases such as atopy and psoriasis or bronchial diseases such as asthma due to the presence of a compound containing a pyrrolidine moiety as a linker.

### Background Art

Skin diseases such as atopic dermatitis and psoriasis have recently emerged as social problems. Particularly, atopic dermatitis is difficult to fundamentally treat and, in many cases, extends to asthma due to its very numerous causes. Unbearable itching (cutaneous pruritus) accompanying these skin diseases increase the number of scratches, leading to further aggravation of symptoms. That is, the symptoms of these skin diseases can be greatly alleviated simply by controlling itching. Thus, many studies aimed at controlling itching are currently underway.

Histamine secreted from mast cells has been found to be the main cause of itching. Recently, cytokines secreted from keratinocytes have also been known to cause itching. The cytokines are associated with thymic stromal lymphopoietin (TSLP) secreted from keratinocytes and stimulate subcutaneous sensory nerves to cause itching.

Various agents such as antihistamines, steroids (cortisol, prednisolone, methylprednisolone, dexamethasone injections and ointments), and moisturizers have been developed to treat itching and inflammation accompanying skin diseases. However, these therapeutic agents are not very effective. Particularly, steroids cause capillaries to expand and make the skin layer thinner (atrophy), resulting in more severe hypersensitivity reactions. Further, when the use of steroids is stopped, a more severe atopic condition called steroid rebound is caused.

Dupilumab consists of an antibody against IL-4Rα, which is an IL-4/IL-13 receptor, and has been recently developed and used as a therapeutic agent. However, dupilumab is expensive and has been reported that it does not show efficacy in all patients and causes adverse reactions in 30% of patients.

Thus, there is a need to develop more economical and safer therapeutic agents for skin diseases.

(Prior Art Patent Document 1) Korean Patent No. 10-1302222

### Detailed Description of the Invention

### Problems to be Solved by the Invention

The present invention is intended to provide a compound containing a pyrrolidine moiety as a linker that is economical and safe and exhibits efficacy in preventing or treating skin diseases.

The present invention is also intended to provide a pharmaceutical composition including the compound or a pharmaceutically acceptable salt thereof as an active ingredient.

### Means for Solving the Problems

One aspect of the present invention provides a compound represented by Formula 1: 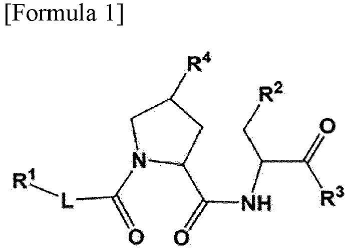 wherein L is selected from the group consisting of a direct bond and C₁-C₅ alkylene, R¹ is C₆-C₁₈ aryl, R² is C₂-C₁₈ heteroaryl, R³ is selected from the group consisting of hydroxyl, hydroxyamino, amino, C₁-C₁₀ monoalkyl, and C₁-C₁₀ dialkylamino, and R⁴ is selected from the group consisting of hydrogen, hydroxyl, and C₁-C₁₀ alkyl, with the proviso that aryl as R¹, heteroaryl as R², monoalkyl or dialkylamino as R³, and alkyl as R⁴ are each independently unsubstituted or substituted with one or more substituents selected from the group consisting of hydroxyl, amino, C₁-C₁₀ alkyl, C₁-C₁₀ alkyloxy, and C₆-C₁₈ aryl, or a pharmaceutically acceptable salt thereof.

The present invention also provides a pharmaceutical composition including the compound or pharmaceutically acceptable salt thereof as an active ingredient.

### Effects of the Invention

The compound of the present invention contains a pyrrolidine moiety as a linker and can efficiently reduce the expression of major inflammatory cytokine factors (e.g., thymic stromal lymphopoietin (TSLP), interleukin (IL), and granulocyte colony stimulating factor (G-CSF)) causing skin diseases such as atopic dermatitis and psoriasis.

The composition of the present invention is economical and safe and can effectively prevent or treat skin diseases due to the presence of the compound or pharmaceutically acceptable salt thereof.

### Brief Description of the Drawings

Fig. 1 shows the results of HPLC analysis for a compound synthesized in Example 1.
Fig. 2 shows the results of HPLC analysis for a compound synthesized in Example 10.
Fig. 3 shows the results of HPLC analysis for a compound synthesized in Example 18.
Fig. 4 shows the results of HPLC analysis for a compound synthesized in Example 19.
Fig. 5 shows the results of HPLC analysis for a compound synthesized in Example 15.
Figs. 6 to 12 are reference images and graphs explaining the results obtained in Experimental Examples 1-4.

### Mode for Carrying out the Invention

It should be understood that the terms and words used in the specification and the claims are not to be construed as having common and dictionary meanings but are construed as having meanings and concepts corresponding to the technical spirit of the present invention in view of the principle that the inventor can define properly the concept of the terms and words in order to describe his/her invention with the best method.

The present inventors have synthesized various compounds and conducted many experiments to develop therapeutic agents for treatment of the respiratory diseases or the skin conditions such as atopic dermatitis and psoriasis or bronchial diseases such as asthma that are safe to humans and can be provided in an economical manner, and as a result, found that a compound containing a pyrrolidine moiety as a linker can inhibit the expression of inflammatory cytokine factors causing skin diseases. The present invention has been accomplished based on this finding. A detailed description of the present invention is as follows.

The present invention provides a compound containing a pyrrolidine moiety as a linker, represented by Formula 1: 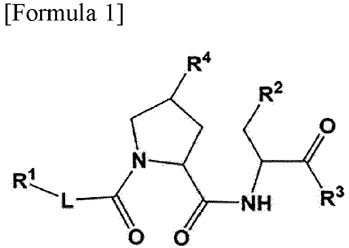 wherein L is selected from the group consisting of a direct bond and C₁-C₅ alkylene, R¹ is C₆-C₁₈ aryl, R² is C₂-C₁₈ heteroaryl, R³ is selected from the group consisting of hydroxyl, hydroxyamino, amino, C₁-C₁₀ monoalkyl, and C₁-C₁₀ dialkylamino, and R⁴ is selected from the group consisting of hydrogen, hydroxyl, and C₁-C₁₀ alkyl, with the proviso that the hydrogen atoms of aryl as R¹, heteroaryl as R², monoalkyl or dialkylamino as R³, and alkyl as R⁴ are each independently unsubstituted or substituted with one or more substituents selected from the group consisting of hydroxyl, amino, C₁-C₁₀ alkyl, C₁-C₁₀ alkyloxy, and C₆-C₁₈ aryl, or a pharmaceutically acceptable salt thereof.

Specifically, the compound of Formula 1 may be represented by Formula 2: 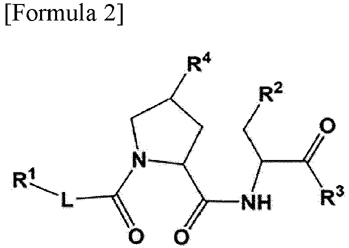 wherein R² to R⁴ and L are as defined in Formula 1 and R⁵ to R⁹ are the same as or different from each other and are each independently selected from the group consisting of hydrogen, hydroxyl (-OH), C₁-C₅ alkyloxy, and C₁-C₅ alkyl, with the proviso that at least one of R⁵ to R⁹ is optionally hydroxyl.

Specifically, the compound of Formula 2 may be represented by one of Formulas 3 to 6: 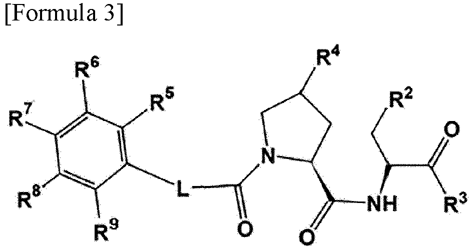
wherein R² to R⁹ and L are as defined in Formula 2, 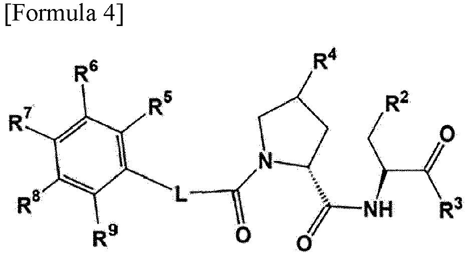
wherein R² to R⁹ and L are as defined in Formula 2, 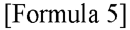 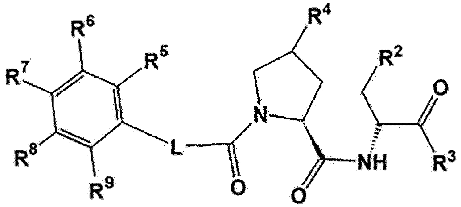
wherein R² to R⁹ and L are as defined in Formula 2, 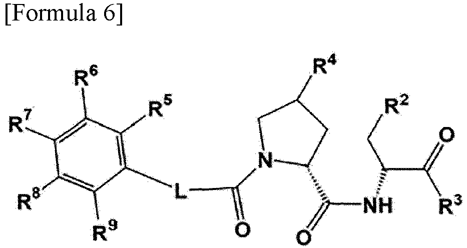
wherein R² to R⁹ and L are as defined in Formula 2.

More specifically, in Formula 1, R² may be imidazolyl, R³ may be hydroxyl, hydroxyamino or amino. R⁴ may be hydrogen or hydroxyl, R⁵ to R⁹ may be each independently hydrogen, hydroxyl or methoxy, and L may be a direct bond, methylene or ethylene.

The compound of Formula 1 may be selected from the group consisting of the following compounds:

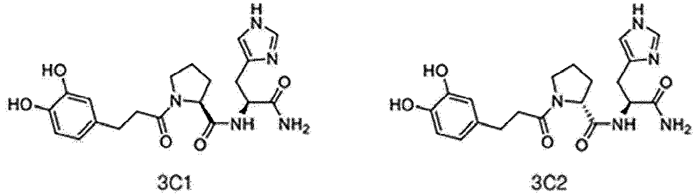

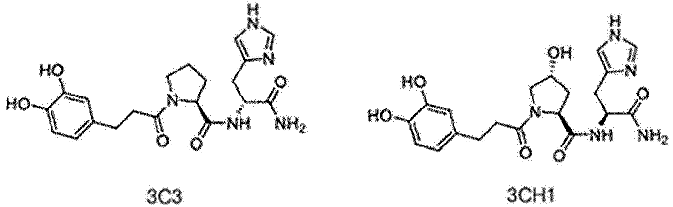

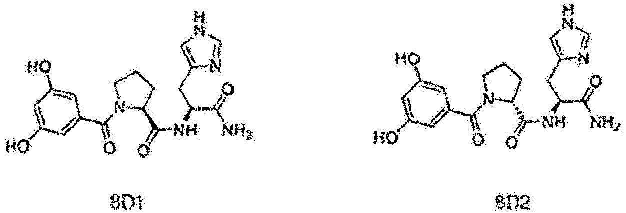

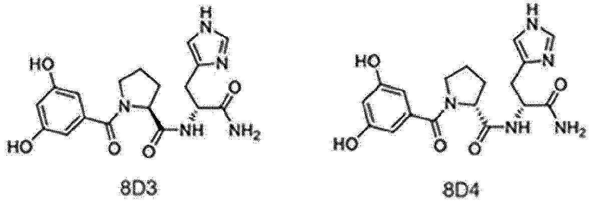

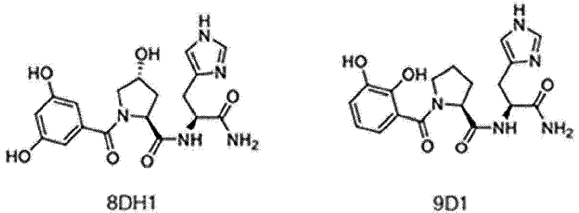

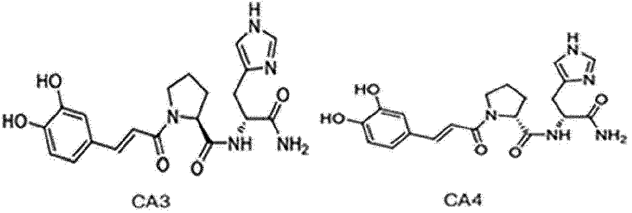

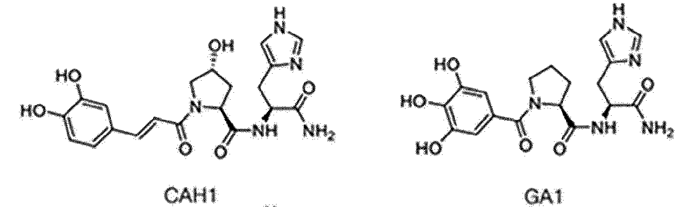

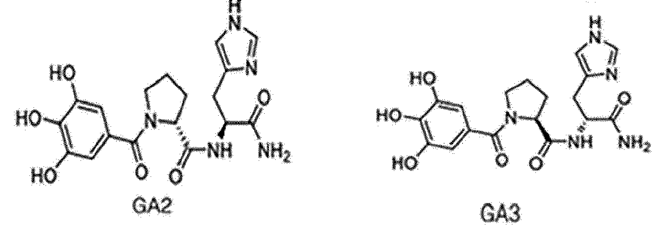

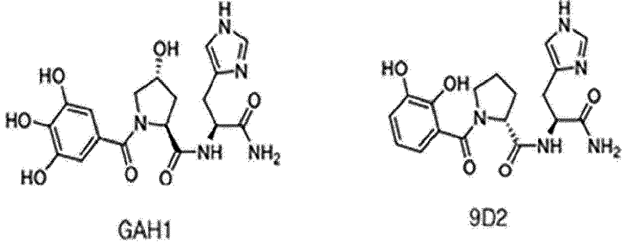

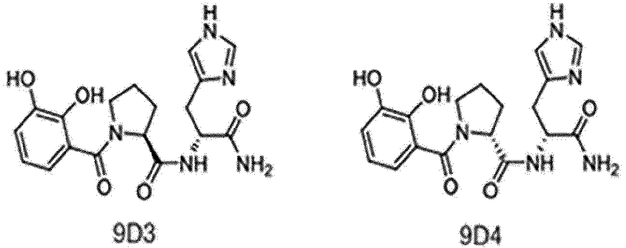

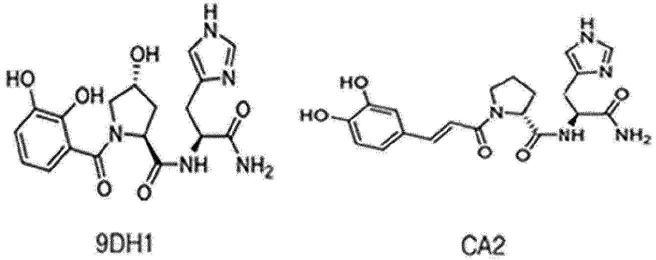

The above compounds enable more efficient control over the expression of inflammatory cytokine factors.

As used herein, the term "alkylene" refers to a divalent functional group. Specific examples of such alkylene groups include methylene, ethylene, propylene, butylene, and pentylene.

As used herein, the term "alkyl" refers to a linear, branched or cyclic functional group. Specific examples of such alkyl groups include methyl, ethyl, propyl, butyl, pentyl, hexyl, heptyl, octyl, nonyl, and decyl.

As used herein, the term "alkyloxy" refers to a monovalent functional group. Specific examples of such alkyloxy groups include methoxy, ethoxy, propoxy, and butoxy.

As used herein, the term "aryl" refers to a monovalent functional group. Specific examples of such aryl groups include phenyl, biphenyl, naphthyl, triphenyl, anthryl, phenanthryl, and indenyl.

As used herein, the term "heteroaryl" refers to a monovalent functional group containing one or more heteroatoms such as N, O, and S atoms. Specific examples of such heteroaryl groups include furanyl, thiophenyl, pyrrolyl, imidazolyl, pyrazolyl, thiazolyl, oxazolyl, pyridyl, pyrimidinyl, pyrazinyl, and triazinyl.

In the above formulas, " 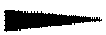 " means a bond protruding forward from the plane of the paper and " 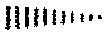 " means a bond protruding backward from the plane of the paper. Unless otherwise specified in the formulas, "-" is meant to include both " 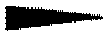 " and " 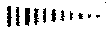 ".

The compound of Formula 1 according to the present invention may be provided in the form of a pharmaceutically acceptable salt. Specifically, the compound of Formula 1 can form an acid addition salt with a pharmaceutically acceptable free acid.

The acid addition salt may be prepared by any suitable method known in the art. Specifically, the acid addition salt may be prepared by dissolving the compound of Formula 1 in an organic solvent such as methanol, ethanol, acetone, methylene chloride or acetonitrile, adding an organic or inorganic acid to the solution, collecting the precipitate by filtration, and drying the precipitate. The organic solvent and the excess acid may be distilled off under reduced pressure, followed by drying and crystallization from an organic solvent.

The free acid may be an organic or inorganic acid. Specifically, the inorganic acid may be hydrochloric acid, phosphoric acid, sulfuric acid, nitric acid or tartaric acid and the organic acid may be methanesulfonic acid, p-toluenesulfonic acid, acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid or vanillic acid.

There is no particular restriction as to the method for preparing the compound of Formula 1 according to the present invention. When considering the production efficiency and purity of the compound, a solid-phase peptide synthesis method using a resin having C-terminal amino groups may be applied to the preparation of the compound of Formula 1.

The resin may be, for example, 2-chlorotrityl chloride resin, Rink amide aminomethyl (AM) resin, Rink amide 4-methylbenzhydrylamine (MBHA) resin or 4-methylbenzhydrylamine (MBHA) resin.

The compound of Formula 1 according to the present invention or pharmaceutically acceptable salt thereof may be used as an active ingredient of a composition effective for preventing or treating skin diseases due to its inhibitory effect on the expression of inflammatory cytokine factors causing skin diseases. Specifically, the cyclic structure of the pyrrolidine linker in Formula 1 makes a hydroxyl group as R¹ adjacent to R² (specifically, an imidazolyl group), which increases the stability of the hydroxyl group. In addition, specific structures in the library of D- and L-stereoisomers having various three-dimensional structures efficiently inhibit the expression of inflammatory cytokine factors, which allows the compound of Formula 1 to exhibit antioxidant activity and effectively prevent or treat skin diseases. The composition effective for preventing or treating skin diseases may be a pharmaceutical composition, a cosmetic composition or a health food composition.

The present invention provides a pharmaceutical composition including the compound of Formula 1 or pharmaceutically acceptable salt thereof as an active ingredient. The content of the active ingredient in the pharmaceutical composition of the present invention may be 0.002 to 0.5 parts by weight, specifically 0.01 to 0.4 parts by weight, based on 100 parts by weight of the pharmaceutical composition.

The compound of Formula 1 or pharmaceutically acceptable salt thereof as an active ingredient of the pharmaceutical composition according to the present invention can inhibit the expression of thymic stromal lymphopoietin (TSLP), interleukin (IL) or granulocyte colony stimulating factor (G-CSF). This ability makes the pharmaceutical composition of the present invention suitable for use as a therapeutic agent for skin diseases. The skin diseases can be selected from the group consisting of atopic dermatitis, eczema, psoriasis, contact dermatitis, and cutaneous pruritus.

The pharmaceutical composition of the present invention can exhibit prophylactic or therapeutic efficacy not only for skin diseases but also for bronchial diseases such as asthma.

The pharmaceutical composition of the present invention may further include at least one carrier or excipient known in the art.

The carrier may be appropriately selected according to the dosage form of the pharmaceutical composition. Specifically, when the pharmaceutical composition is intended for oral administration, the carrier may be cellulose, calcium silicate, corn starch, lactose, sucrose, dextrose, calcium phosphate, stearic acid, magnesium stearate, calcium stearate, gelatin or talc. Alternatively, the pharmaceutical composition may be intended for parenteral administration. In this case, the carrier may be, for example, water, saline, aqueous glucose solution, aqueous similar sugar solution, alcohol, glycol, ether, oil, fatty acid, fatty acid ester or glyceride.

The excipient may be selected from the group consisting of sweeteners, binders, solubilizers, dissolution aids, wetting agents, emulsifiers, isotonic agents, adsorbents, disintegrants, antioxidants, preservatives, lubricants, fillers, flavoring agents, and mixtures thereof. Specific examples of suitable excipients include lactose, dextrose, sucrose, mannitol, sorbitol, cellulose, glycine, silica, magnesium aluminum silicate, starch, gelatin, gum tragacanth, alginic acid, sodium alginate, methylcellulose, sodium carboxymethyl cellulose, water, ethanol, polyethylene glycol, polyvinylpyrrolidone, sodium chloride, and calcium chloride.

The pharmaceutical composition of the present invention may be formulated into conventional oral preparations such as pills, powders, granules, tablets, capsules, suspensions, emulsions, syrups, and aerosols or parenteral preparations such as external preparations, suppositories, and sterile injectable solutions.

The dose of the pharmaceutical composition according to the present invention administered to a human may be determined depending on the general health, age, body weight, and sex of the subject or the dosage form of the composition. The pharmaceutical composition of the present invention may be administered once a day or in divided doses several times daily.

The pharmaceutical composition of the present invention may be a cosmetic composition. In this case, the cosmetic composition is effective in preventing skin diseases due to the presence of the compound of Formula 1 or pharmaceutically acceptable salt thereof as an active ingredient.

The content of the active ingredient in the cosmetic composition of the present invention may be 0.001 to 0.5 parts by weight, specifically 0.01 to 0.4 parts by weight, based on 100 parts by weight of the cosmetic composition.

The cosmetic composition of the present invention may further include at least one additive commonly used in the art. Examples of such additives include purified water, antioxidants, stabilizers, pigments, functional additives, and fragrances.

The cosmetic composition of the present invention may be formulated into conventional preparations. Specific examples of the preparations include solutions, suspensions, emulsions, pastes, gels, creams, lotions, powders, and sprays.

The pharmaceutical composition of the present invention may be a health food composition. In this case, the health food composition is effective in preventing skin diseases due to the presence of the compound of Formula 1 or pharmaceutically acceptable salt thereof as an active ingredient.

The health food composition of the present invention may further include at least one additive commonly used in the art. Examples of such additives include flavoring agents, natural carbohydrates, minerals (electrolytes), flavorants, colorants, thickeners, pH adjusting agents, stabilizers, preservatives, glycerin, alcohol, and carbonating agents. The natural carbohydrates may be, for example, glucose, fructose, maltose, sucrose, dextrin, cyclodextrin, xylitol, sorbitol, and erythritol.

The flavoring agents may be, for example, thaumatin, rebaudioside A, glycyrrhizin, saccharin, and aspartame.

The health food composition of the present invention may be formulated into conventional preparations such as capsules, tablets, powders, liquids, pills, pastes, syrups, gels, and jellies.

The present invention will be more specifically explained with reference to the following examples. However, these examples are provided for illustrative purposes and do not serve to limit the scope of the invention. It will be obvious to those skilled in the art that various modifications and changes are possible without departing from the scope and spirit of the invention.

### [Example 1]

5 ml of dimethylformamide was placed in a reaction vessel into which 417 mg of an Fmoc-suberol MBHA resin (substitution rate: 0.48 mmol/g, 0.2 mmol scale) had been introduced. After the resin was allowed to swell for 5 min, the mixture was filtered and 7 ml of a 20% piperidine solution was added thereto. The reaction was allowed to proceed for 5 min to remove the N-terminal protecting groups (Fmoc-), followed by filtration. Then, dimethylformamide (3 times, 1 min for each wash), methanol (once, 1 min), and dimethylformamide (3 times, 1 min for each wash) were sequentially added (each 7 ml) to wash the resin, followed by filtration. Next, 3 ml (0.6 mmol, 3 equiv.) of a 0.2 M Fmoc-His(Trt)-OH solution, 3 ml (0.6 mmol, 3 equiv.) of a 0.2 M O-(6-chlorobenzotriazol-1-yl)-N,N,N',N'-tetramethyluronium hexafluorophosphate (HCTU) solution, and 3 ml (3.0 mmol, 15 equiv.) of a 1.0 M diisopropylethylamine solution were added to the resin. The reaction was allowed to proceed under nitrogen gas bubbling at room temperature for 1 h. The resulting reaction solution was filtered to obtain Resin 1 where the reaction had been completed. Resin 1 was washed with dimethylformamide (3 times, 1 min for each wash), methanol (once, 1 min), and dimethylformamide (three times, each time for 1 min).

7 ml of a 20% piperidine solution was added to washed Resin 1. The reaction was allowed to proceed for 5 min to remove the N-terminal protecting groups (Fmoc-), followed by filtration and washing. Next, 3 ml (0.6 mmol, 3 equiv.) of a 0.2 M Fmoc-Pro-OH solution, 3 ml (0.6 mmol, 3 equiv.) of a 0.2 M O-(6-chlorobenzotriazol-1-yl)-N,N,N',N'-tetramethyluronium hexafluorophosphate (HCTU) solution, and 3 ml (3.0 mmol, 15 equiv.) of a 1.0 M diisopropylethylamine solution were added to the resin. The reaction was allowed to proceed under nitrogen gas bubbling at room temperature for 1 h. The resulting reaction solution was filtered to obtain Resin 2 where the reaction had been completed. Resin 2 was washed with dimethylformamide (3 times, 1 min for each wash), methanol (once, 1 min), and dimethylformamide (three times, each time for 1 min).

3 ml (0.6 mmol, 3 equiv.) of a 0.2 M 3,4-dihydroxydihydrocinnamic acid solution, 3 ml (0.6 mmol, 3 equiv.) of a 0.2 M O-(6-chlorobenzotriazol-1-yl)-N,N,N',N'-tetramethyluronium hexafluorophosphate (HCTU) solution, and 3 ml (3.0 mmol, 15 equiv.) of a 1.0 M diisopropylethylamine solution were added to washed Resin 2. The reaction was allowed to proceed under nitrogen gas bubbling at room temperature for 1 h. The resulting reaction solution was filtered to obtain Resin 3 where the reaction had been completed. Resin 3 was washed with dimethylformamide (3 times, 1 min for each wash), methanol (once, 1 min), and dimethylformamide (three times, each time for 1 min).

Washed Resin 3 was collected by filtration, washed again with dichloromethane (6 times, 1 min for each wash), and dried under a nitrogen atmosphere for 3 h to obtain Resin 4 to which the compound represented by Formula 4 was bound.

Resin 4 was placed in a 25 ml Libra tube and 10 ml of dichloromethane was added thereto. The resin was allowed to swell for 3 min, followed by filtration. Next, 6 ml of a cleavage cocktail solution (TFA:TIS:H₂O ratio (wt%) = 95:2.5:2.5) was added to the swollen resin. The reaction was allowed to proceed with shaking at room temperature for 3 h. The resulting reaction solution was transferred to a 50 ml conical tube and diethyl ether was added to the 50 ml mark on the tube to obtain a precipitate. Next, the conical tube was shaken with a vortex mixer for 30 sec, followed by centrifugation at 3000 rpm for 3 min. Then, the supernatant was discarded and diethyl ether was added to the 50 ml mark. The resulting precipitate was washed three times, filtered, and dried in a vacuum desiccator for 1 h to give 31.7 mg of the compound represented by 3C1 as a powder (purity: 98.6%, Mw: 415.45 g/mol, ESI-MS m/z: 416.20 [M+H]).

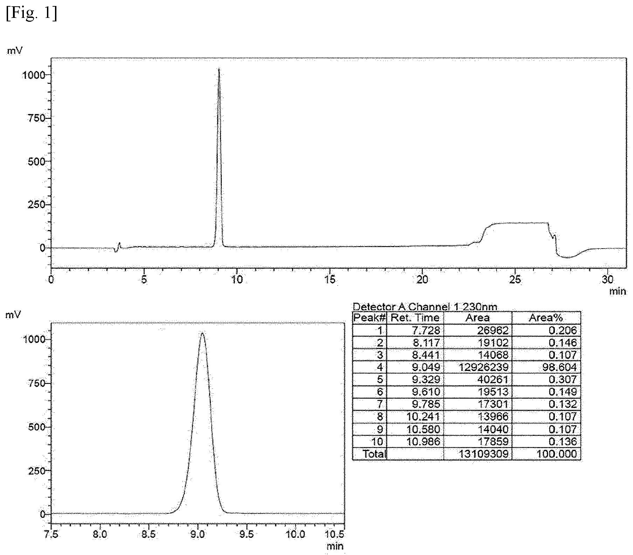

The compound represented by 3C1 was analyzed by high-performance liquid chromatography (HPLC). The results are shown in Fig. 1.

### [Examples 2-22]

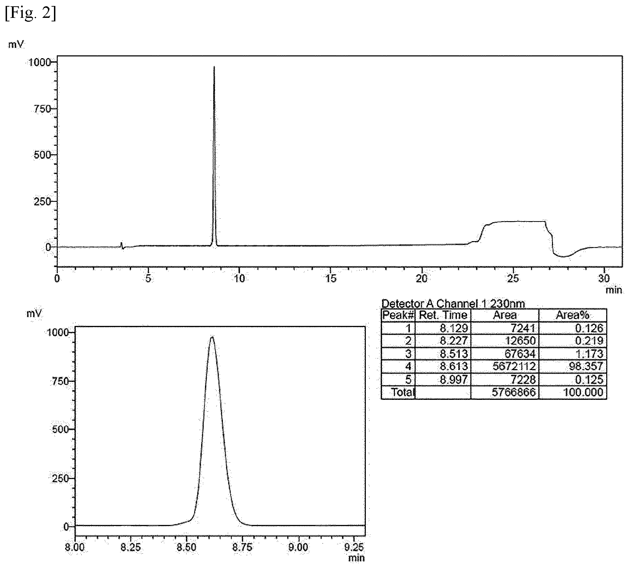

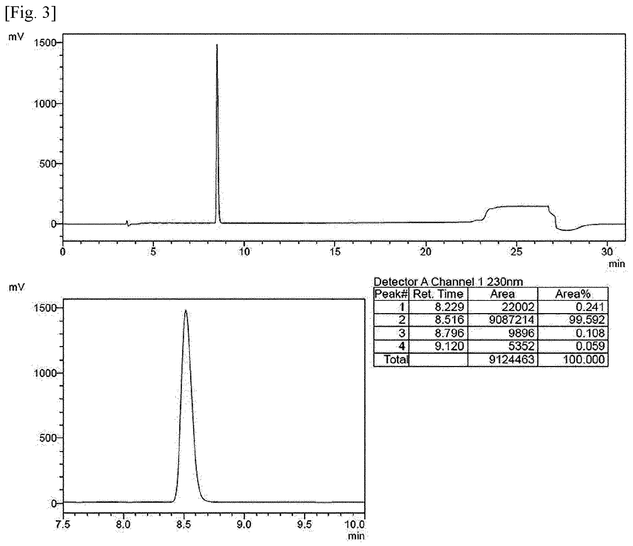

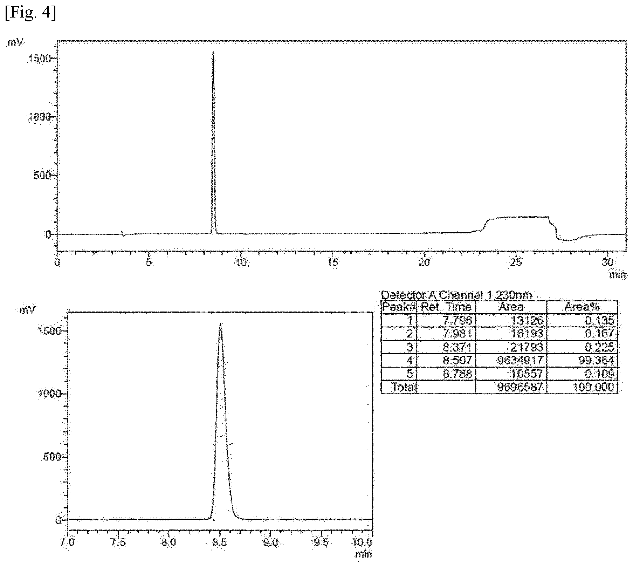

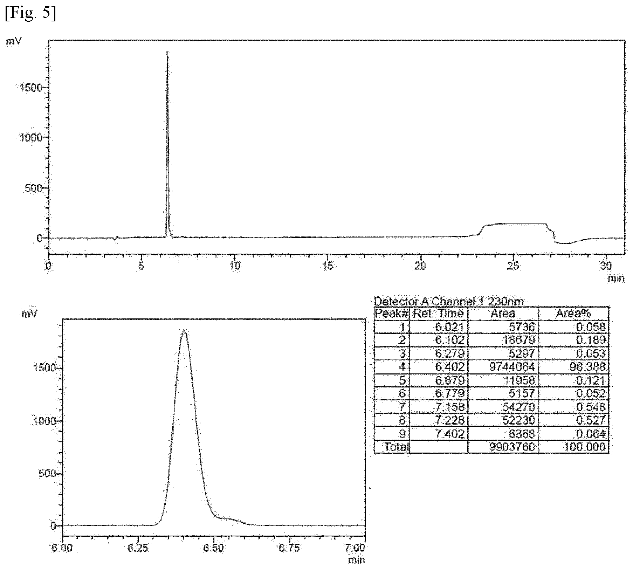

The procedure of Example 1 was repeated except that the reactants were changed as shown in Table 1. The synthesized compounds were used in animal tests. The results of HPLC analysis for representative ones of the compounds are shown in Figs. 2-5.

**[Table 1]**

| NO. | Code | Reactant 1 | Reactant 2 | Reactant 3 | Molecular weight (g/mol) | Purity (%) |
|---|---|---|---|---|---|---|
| Example 1 | 3C1 | Fmoc-His(Trt)-OH solution | Fmoc-Pro-OH solution | 3.4-dihvdroxvdihvdrocinnamic acid solution | 415.45 | 98.6 |
| Example 2 | 3C2 | Fmoc-His(Trt)-OH solution | Fmoc-D-Pro-OH solution | 3,4-dihydroxydihydrocinnamic acid solution | 415.45 | 99.1 |
| Example 3 | 3C3 | Fmoc-D-His(Trt)-OH solution | Fmoc-Pro-OH solution | 3,4-dihydroxydihydrocinnamic acid solution | 415.45 | 98.6 |
| Example 4 | 3CH1 | Fmoc-His(Trt)-OH | Fmoc-Hvp-OH solution | 3,4-dihvdroxvdihvdrocinnamic acid solution | 431.45 | 98.4 |
| Example 5 | 8D1 | Fmoc-His(Trt)-OH solution | Fmoc-Pro-OH solution | 2,3-dihvdroxvbenzoic acid solution | 387.4 | 98.4 |
| Example 6 | 8D2 | Fmoc-His(Trt)-OH solution | Fmoc-D-Pro-OH solution | 2,3-dihydroxybenzoic acid solution | 387.4 | 93.3 |
| Example 7 | 8D3 | Fmoc-D-His(Trt)-OH solution | Fmoc-Pro-OH solution | 2,3-dihydroxybenzoic acid solution | 387.4 | 96.6 |
| Example 8 | 8D4 | Fmoc-D-His(Trt)-OH solution | Fmoc-D-Pro-OH solution | 2.3-dihvdroxvbenzoic acid solution | 387.4 | 98.1 |
| Example 9 | 8DH1 | Fmoc-His(Trt)-OH solution | Fmoc-Hvp-OH solution | 2,3-dihvdroxvbenzoic acid solution | 403.4 | 98.7 |
| Example 10 | 9D1 | Fmoc-His(Trt)-OH solution | Fmoc-Pro-OH solution | 2,3-dihydroxybenzoic acid solution | 387.4 | 98.3 |
| Example 11 | CA3 | Fmoc-D-His(Trt)-OH solution | Fmoc-Pro-OH solution | Caffeic acid | 413.43 | 96.9 |
| Example 12 | CA4 | Fmoc-D-His(Trt)-OH solution | Fmoc-D-Pro-OH solution | Caffeic acid | 413.43 | 96.0 |
| Example 13 | CAH1 | Fmoc-His(Trt)-OH | Fmoc-Hvp-OH solution | Caffeic acid | 429.43 | 95.1 |
| Example 14 | GA1 | Fmoc-His(Trt)-OH | Fmoc-Pro-OH solution | Gallic acid solution | 403.4 | 97.9 |
| Example 15 | GA2 | Fmoc-His(Trt)-OH solution | Fmoc-D-Pro-OH solution | Gallic acid solution | 403.4 | 95.5 |
| Example 16 | GA3 | Fmoc-D-His(Trt)-OH solution | Fmoc-Pro-OH solution | Gallic acid solution | 403.4 | 96.4 |
| Example 17 | GAH1 | Fmoc-His(Trt)-OH solution | Fmoc-Hvp-OH solution | Gallic acid solution | 419.39 | 93.5 |
| Example 18 | 9D2 | Fmoc-His(Trt)-OH solution | Fmoc-D-Pro-OH solution | 2,3-dihydroxybenzoic acid solution | 387.4 | 99.5 |
| Example 19 | 9D3 | Fmoc-D-His(Trt)-OH solution | Fmoc-Pro-OH solution | 2,3-dihydroxybenzoic acid solution | 387.4 | 99.3 |
| Example 20 | 9D4 | Fmoc-D-His(Trt)-OH solution | Fmoc-D-Pro-OH solution | 2.3-dihvdroxvbenzoic acid solution | 387.4 | 99.4 |
| Example 21 | 9DH1 | Fmoc-His(Trt)-OH solution | Fmoc-Hvp-OH solution | 2,3-dihvdroxvbenzoic acid solution | 403.4 | 96.2 |
| Example 22 | CA2 | Fmoc-His(Trt)-OH solution | Fmoc-D-Pro-OH solution | Caffeic acid | 413.43 | 98.2 |

### [Experimental Example 1]

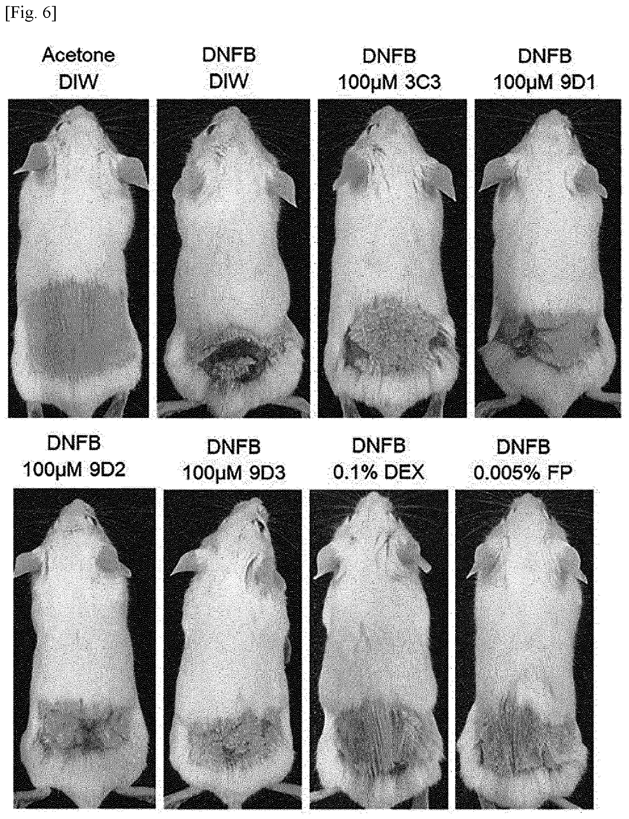

After the abdominal hair of 6-week-old female BALB/C mice was shaved, 100 µL of 0.15% 2,4-dinitrofluorobenzene (DNFB) was applied to the shaved skin to induce symptoms of atopic dermatitis. 7 days later, 100 µL of 0.15% DNFB was applied to the shaved skin of the back every 3 days until 16 days have passed. After 7 days, each of the compounds synthesized in Examples 1-22 (100 µM) was applied to the skin once a day until 16 days had passed. After completion of the application, changes in the skin of the female mice were observed. The results are shown in Fig. 6. Deionized water (DIW), dexamethasone (DEX, 0.1%), and fluticasone propionate (FP, 0.005%) as controls were applied to the skin of the female mice.

Referring to Fig. 6, the inventive compounds synthesized in Examples 1-22 were confirmed to be effective in atopic dermatitis to a level equal to or higher than those achieved by the positive controls.

### [Experimental Example 2]

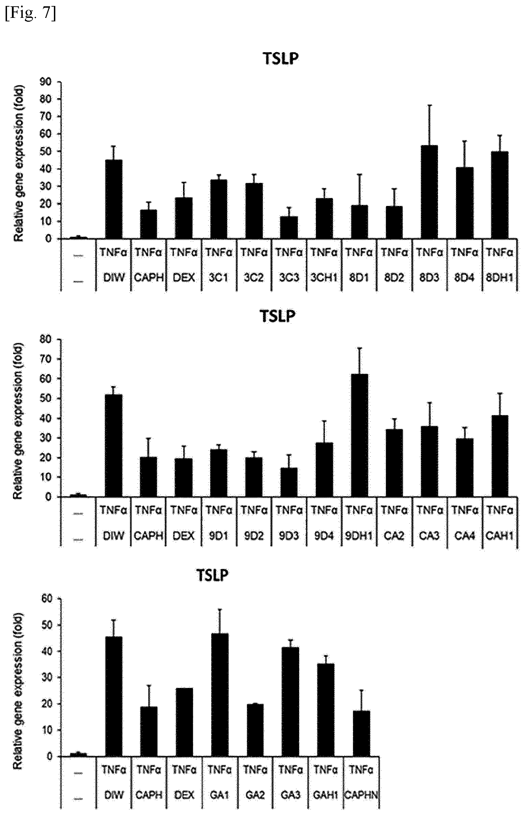

Human HaCaT keratinocytes were cultured in Dulbecco's modified Eagle's medium (DMEM) supplemented with 10% fetal bovine serum (FBS), 100 units/ml penicillin, and 100 µg/ml streptomycin at 37 °C while maintaining a proper humidity level and 5% CO₂. Next, cells were subcultured in 12 wells and the medium was treated with 50 ng/ml tumor necrosis factor α (TNFα) to induce the expression of TSLP. After 1 h, each of the compounds synthesized in Examples 1-22 (1 µM) was added to the medium, followed by culture for another 1 h. After completion of the culture, real time PCR was performed to determine whether the expression of TSLP causing atopic dermatitis was increased or decreased. The results are shown in Fig. 7. Deionized water (DIW) and dexamethasone (DEX, 200 nM) were used as controls.

For real time PCR, RNA was extracted using RNAiso Plus (TAKARA, Japan) and cDNA was synthesized using a cDNA synthesis kit (TAKARA, JAPAN). The polymerization reaction was carried out using the synthesized cDNA and a Sybr green kit (Enzynomics, Korea). Here, the following primers were used: human RPLP0 forward, 5'-AGC CCA GAA CAC TGG TCT C-3', reverse, 5'-ACT CAG GAT TTC AAT GGT GCC-3', human TSLP forward, 5'-AAT CCA GAG CCT AAC CTT CAA TC-3', reverse, 5'-GTA GCA TTT ATC TGA GTT TCC GAA TA-3', mouse RPLP0 forward, 5'-AGA TTC GGG ATA TGC TGT TGG C-3', reverse, 5'-TCG GGT CCT AGA CCA GTG TTC-3', mouse TSLP forward, 5'-AGC TTG TCT CCT GAA AAT CGA G-3', reverse 5'-AGG TTT GAT TCA GGC AGA TG TT-3', mouse CSF2 forward 5'-AGG GTC TAC GGG GCA ATT TC-3', reverse 5'-TCA CAG TCC GTT TCC GGA GTT-3', mouse IL-4 forward, 5'-GGT CTC AAC CCC CAG CTA GT-3', reverse, 5'-GCC GAT GAT CTC TCT CAA GTG AT-3', mouse IL-10 forward, 5'-GCT CTT ACT GAC TGG CAT GAG-3', reverse, 5'-CGC AGC TCT AGG AGC ATG TG-3', mouse IL-13 forward, 5'-CCT GGC TCT TGC TTG CCT T-3', reverse, 5'-GGT CTT GTG TGA TGT TGC TCA-3', mouse IL-17 forward 5'-GCT GAC CCC TAA GAA ACC CC-3', reverse, 5'-GAA GCA GTT TGG GAC CCC TT-3', mouse IL-22 forward 5'-ATG AGT TTT TCC CTT ATG GGG AC-3', reverse 5'-GAA GCA GTT TGG GAC CCC TT-3', mouse IL-25 forward, 5'-ACA GGG ACT TGA ATC GGG TC-3', reverse 5'-TGG TAA AGT GGG ACG GAG TTG-3', mouse IL-31 forward 5'-TCA GCA GAC GAA TCA ATA CAG C-3', reverse 5'-TCG CTC AAC ACT TTG ACT TTC T-3', mouse IL-33 forward 5'-GCT GCA GAA GGG AGA AAT CAC G-3', reverse 5'-GAG TTG GAA TAC TTC ATT CTA GGT CTC A-3'.

Referring to Fig. 7, the use of the inventive compounds synthesized in Examples 1-22 was confirmed to reduce the expression of TSLP (TSLP mRNA), supporting that the inventive compounds inhibit the expression of TSLP causing atopic dermatitis.

### [Experimental Example 3]

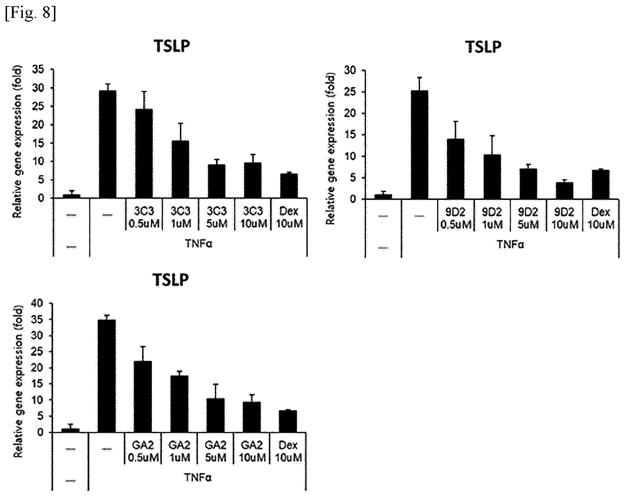

The procedure of Experimental Example 2 was repeated except that the concentrations of the compounds synthesized in Examples 3, 15, and 18 were changed to determine whether the compounds increase or decrease the expression of TSLP causing atopic dermatitis. The results are shown in Fig. 8. Dexamethasone (DEX, 10 µM) was used as a control.

Referring to Fig. 8, the use of the inventive compounds was confirmed to reduce the expression of TSLP (TSLP mRNA) in a concentration dependent manner.

### [Experimental Example 4]

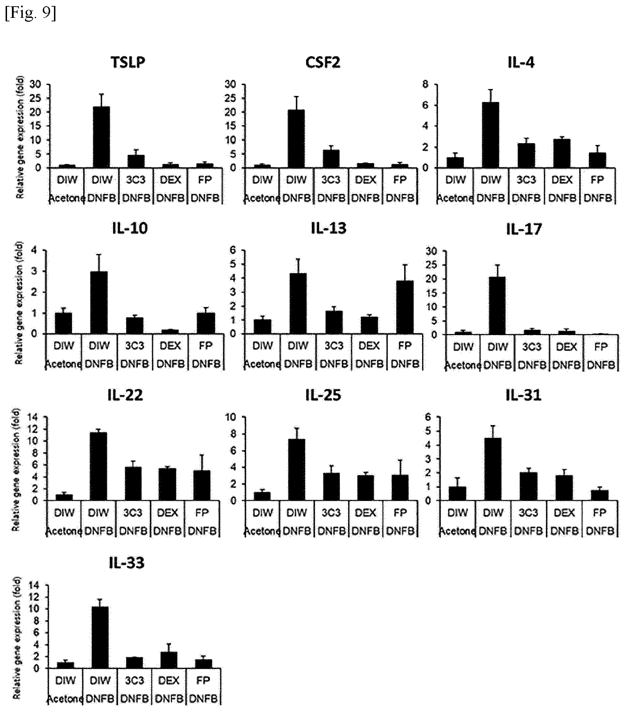

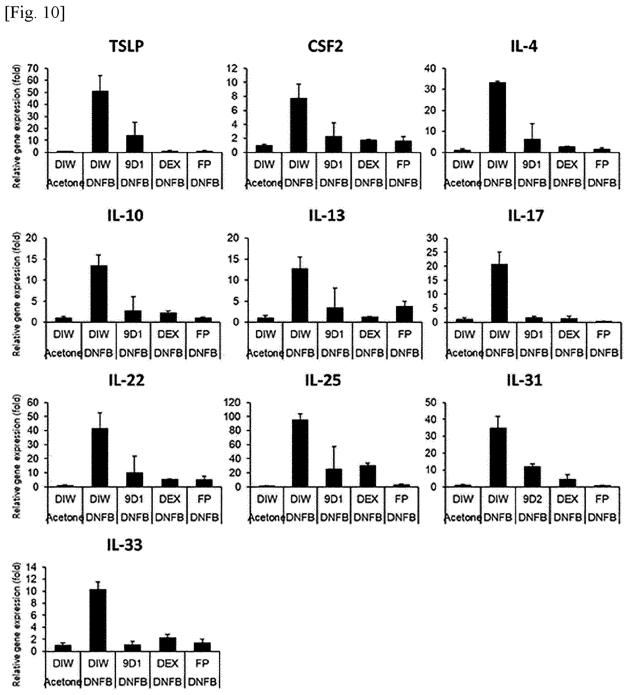

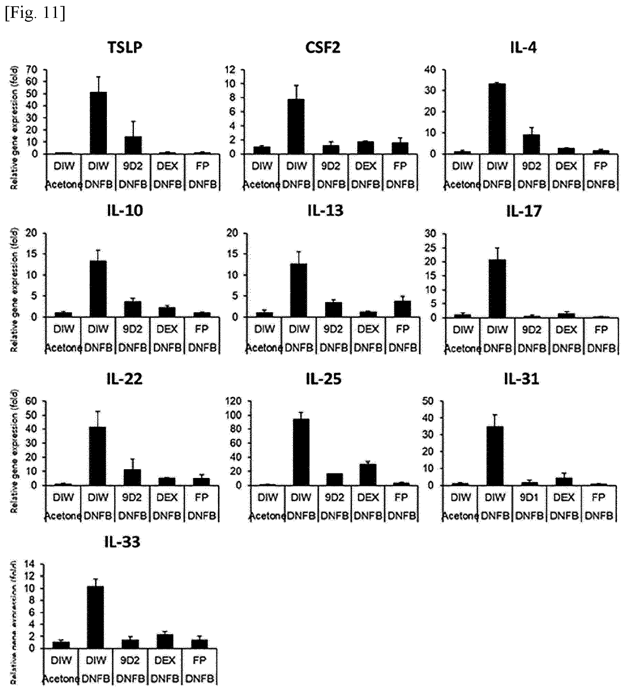

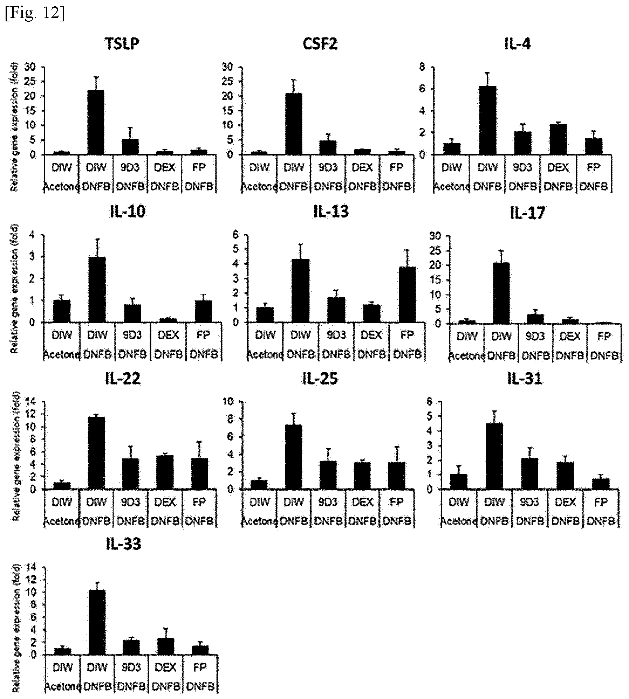

After the abdominal hair of 6-week-old female BALB/C mice was shaved, 100 µL of 0.15% 2,4-dinitrofluorobenzene (DNFB) was applied to the shaved skin to induce symptoms of atopic dermatitis. 7 days later, 100 µL of 0.15% DNFB was applied to the shaved skin of the back every 3 days until 16 days have passed. After 7 days, each of the compounds synthesized in Examples 1-22 (100 µM) was applied to the skin once a day until 16 days had passed. After completion of the application, RNA was extracted from the skin of the female mice. The real time PCR procedure described in Experimental Example 2 was used to determine whether the expression of factors (genes) causing atopic dermatitis was increased or decreased. The results are shown in Figs. 9 to 12. Deionized water (DIW), dexamethasone (DEX), and fluticasone propionate (FP) as controls were applied to the skin of the female mice.

Referring to Figs. 9 to 12, the use of the inventive compounds synthesized in Examples 3, 10, 18, and 19 was confirmed to reduce the expression of the factors (genes), supporting that the inventive compounds inhibit the expression of factors (genes) causing atopic dermatitis.

### [Experimental Example 3]

The procedure of Experimental Example 2 was repeated except that the concentrations of the compounds synthesized in Examples 3, 15, and 18 were changed to determine whether the compounds increase or decrease the expression of TSLP causing atopic dermatitis. The results are shown in Fig. 8. Dexamethasone (DEX, 10 µM) was used as a control.

Referring to Fig. 8, the use of the inventive compounds was confirmed to reduce the expression of TSLP (TSLP mRNA) in a concentration dependent manner.

### [Experimental Example 4]

After the abdominal hair of 6-week-old female BALB/C mice was shaved, 100 µL of 0.15% 2,4-dinitrofluorobenzene (DNFB) was applied to the shaved skin to induce symptoms of atopic dermatitis. 7 days later, 100 µL of 0.15% DNFB was applied to the shaved skin of the back every 3 days until 16 days have passed. After 7 days, each of the compounds synthesized in Examples 1-22 (100 µM) was applied to the skin once a day until 16 days had passed. After completion of the application, RNA was extracted from the skin of the female mice. The real time PCR procedure described in Experimental Example 2 was used to determine whether the expression of factors (genes) causing atopic dermatitis was increased or decreased. The results are shown in Figs. 9 to 12. Deionized water (DIW), dexamethasone (DEX), and fluticasone propionate (FP) as controls were applied to the skin of the female mice.

Referring to Figs. 9 to 12, the use of the inventive compounds synthesized in Examples 3, 10, 18, and 19 was confirmed to reduce the expression of the factors (genes), supporting that the inventive compounds inhibit the expression of factors (genes) causing atopic dermatitis.

## Claims

1. A compound represented by Formula 1: 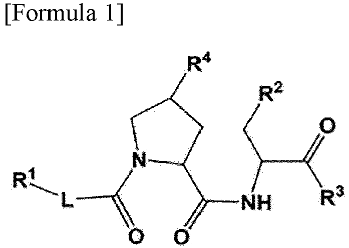 wherein L is selected from the group consisting of a direct bond and C₁-C₅ alkylene, R¹ is C₆-C₁₈ aryl, R² is C₂-C₁₈ heteroaryl, R³ is selected from the group consisting of hydroxyl, hydroxyamino, amino, C₁-C₁₀ monoalkyl, and C₁-C₁₀ dialkylamino, and R⁴ is selected from the group consisting of hydrogen, hydroxyl, and C₁-C₁₀ alkyl, with the proviso that aryl as R¹, heteroaryl as R², monoalkyl or dialkylamino as R³, and alkyl as R⁴ are each independently unsubstituted or substituted with one or more substituents selected from the group consisting of hydroxyl, amino, C₁-C₁₀ alkyl, C₁-C₁₀ alkyloxy, and C₆-C₁₈ aryl, or a pharmaceutically acceptable salt thereof.

2. The compound or pharmaceutically acceptable salt thereof according to claim 1, wherein the compound of Formula 1 is represented by Formula 2: 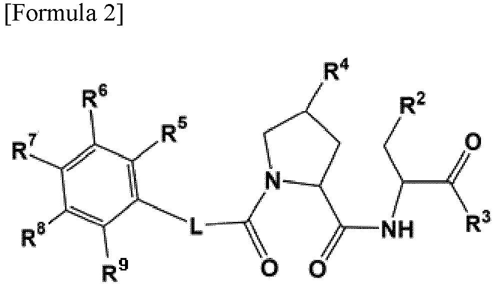 wherein R² to R⁴ and L are as defined in claim 1 and R⁵ to R⁹ are the same as or different from each other and are each independently selected from the group consisting of hydrogen, hydroxyl, C₁-C₅ alkyloxy, and C₁-C₅ alkyl, with the proviso that at least one of R⁵ to R⁹ is optionally hydroxyl.

3. The compound or pharmaceutically acceptable salt thereof according to claim 1, wherein the compound of Formula 1 is represented by one of Formulas 3 to 6: 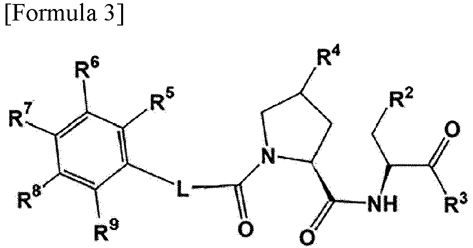 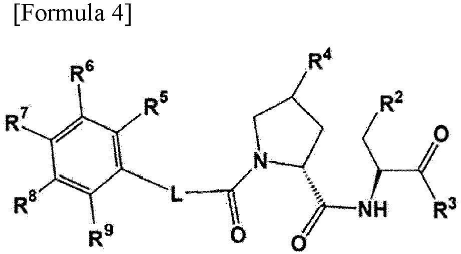 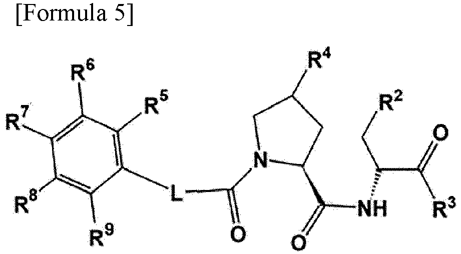 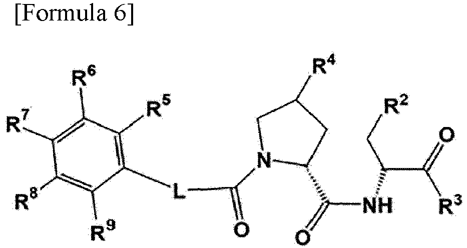 wherein R2 to R9 and L are as defined in claim 1. R5 to R9 are the same as or different from each other and are each independently selected from the group consisting of hydrogen, hydroxyl, C1-C5 alkyloxy, and C1-C5 alkyl, with the proviso that at least one of R⁵ to R9 is optionally hydroxyl.

4. The compound or pharmaceutically acceptable salt thereof according to claim 1, wherein R² is imidazolyl.

5. The compound or pharmaceutically acceptable salt thereof according to claim 1, wherein R³ is amino.

6. The compound or pharmaceutically acceptable salt thereof according to claim 1, wherein L is selected from the group consisting of a direct bond, methylene, and ethylene.

7. A pharmaceutical composition comprising the compound or pharmaceutically acceptable salt thereof according to any one of claims 1 to 6 as an active ingredient.

8. The pharmaceutical composition according to claim 7, wherein the compound or pharmaceutically acceptable salt thereof inhibits the expression of thymic stromal lymphopoietin (TSLP), interleukin (IL) or granulocyte colony stimulating factor (G-CSF).

9. The pharmaceutical composition according to claim 7, wherein the composition is for the prevention and treatment of the respiratory diseases or the skin conditions that are selected from the group consisting of atopic dermatitis, eczema, psoriasis, contact dermatitis, and cutaneous pruritus.
